# FASCICULE DE BREVET EUROPEEN

(11) **EP 1 278 718 B1**
(45) Date de publication et mention de la délivrance du brevet: **17.12.2003**
(21) Numéro de dépôt: 01925635.3
(22) Date de dépôt: 12.04.2001
(51) Int. Cl.: C07C 233/51, C07C 311/12, C07C 231/02, A61K 7/42

(54) **NOUVEAUY DERIVES D'AMINOACIDES, LEUR PROCEDE DE PREPARATION, COMPOSITIONS LES CONTENANT ET LEURS UTILISATIONS COMME FILTRES SOLAIRES**
NEUE AMINSÄURE-DERIVATE, VERFAHREN ZUR IHRER HERSTELLUNG, IHRE ZUSAMMENSETZUNGEN UND IHRE VERWENDUNG ALS SONNENFILTER
NOVEL AMINO ACID DERIVATIVES, PREPARATION METHOD, COMPOSITIONS CONTAINING SAME AND USES THEREOF AS SUNSCREENS

(30) Priorité: 27.04.2000 FR 0005393
(43) Date de publication de la demande: 29.01.2003
(73) Titulaire: L'OREAL, 75008 Paris (FR)
(72) Inventeur: BORDIER, Thierry, F-93290 Tremblay (FR); PHILIPPE, Michel, F-91320 Wissous (FR)
(74) Mandataire: Miszputen, Laurent
(86) Numéro de dépôt international: FR0101137
(87) Numéro de publication internationale: WO01081297

(56) Documents cités:
- EP-A- 0 554 898
- US-A- 5 004 594
- MASATO NANASAWA; HIROYOSHI KAMOGAWA: "Syntheses of Photosensitive Poly(amino acids)" BULLETIN OF THE CHEMICAL SOCIETY OF JAPAN., vol. 48, no. 9, 1975, pages 2588-91, XP000960601 JAPAN PUBLICATIONS TRADING CO. TOKYO., JP ISSN: 0009-2673
- R ANDRUSZKIEWICZ ET AL: "Structural determinants of inhibitory activity of N3-(4-methoxyfumaroyl) -L-2,3-diaminopropanoic acid towards glucosamine-6-phosphate synthase" POLISH JOURNAL OF CHEMISTRY., vol. 67, 1993, pages 673-83, XP000964579 POLISH CHEMICAL SOCIETY., XX

## Description

La présente invention concerne de nouveaux dérivés d'amino-acides, leur procédé de préparation et leurs utilisations en tant que filtres UV, notamment dans le domaine cosmétique.

La présente invention concerne également l'utilisation de ces nouveaux composés pour la protection de la peau et/ou des cheveux contre le rayonnement ultraviolet, ou pour la protection de toute autre matière sensible aux UV (verres minéraux ou organiques, plastiques, produits alimentaires, peintures ou autre).

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Ainsi, dans le but d'assurer une protection de la peau et des cheveux contre l'ensemble du rayonnement UV qui soit la plus complète et la plus efficace possible, on utilise généralement dans la fabrication des compositions antisolaires des associations de filtres actifs dans l'UV-A et de filtres actifs dans l'UV-B.

On connaît dans l'art antérieur et en particulier dans le brevet US 5,004,594 des composés benzylidènecampre sulfonamides dérivés d'aminoacides en tant que filtres UV, notamment dans le domaine cosmétique. On connaît également dans le brevet FR 2 579 461 d'autres filtres solaires dérivés d'aminoacides à savoir des composés amides de l'acide para-méthoxycinnamique et de l'acide urocanique. Ces dérivés d 'aminoacides sont capables d'absorber dans l'UV-B et/ou l'UV-A. Ils sont solubles dans les solvants couramment utilisés dans les formulations solaires et notamment les huiles. Ils ne présentent aucune activité de diffusion de la lumière.

Or, à la suite d'importantes recherches menées dans le domaine de la photoprotection évoqué ci-dessus, la Demanderesse a maintenant découvert de façon surprenante des nouveaux dérivés aminoacides, insolubles ou susbstantiellement insolubles dans l'eau et les solvants organiques couramment utilisés, capables à la fois d'absorber et de diffuser les radiations UV dans les formulations solaires. La Demanderesse a constaté que les dérivés aminoacides conformes à l'invention permettaient d'obtenir par rapport aux dérivés d'aminoacides connus une photoprotection plus renforcée.

Cette découverte est à la base de l'invention.

Ainsi, la présente invention a pour objet une composition destinée à protéger une matière sensible au rayonnement ultraviolet; en particulier au rayonnement solaire, comprenant une quantité efficace d'au moins un composé de formule (I) suivante : 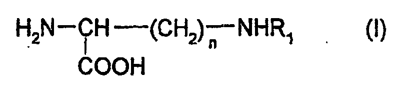 dans laquelle :
- n est un nombre entier de 1 à 4
- le radical R₁ désigne l'un des groupes de formule (1), (2) ou (3) (4), (5) (6) ou (7) suivants : 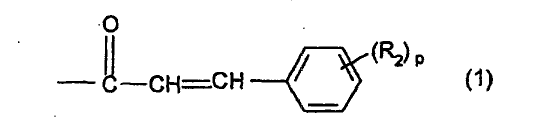 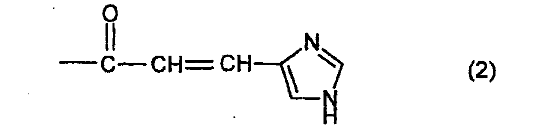 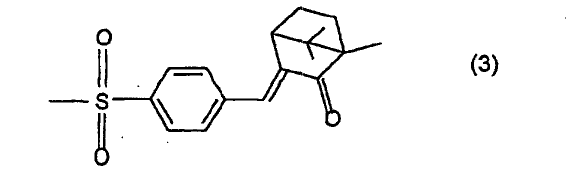 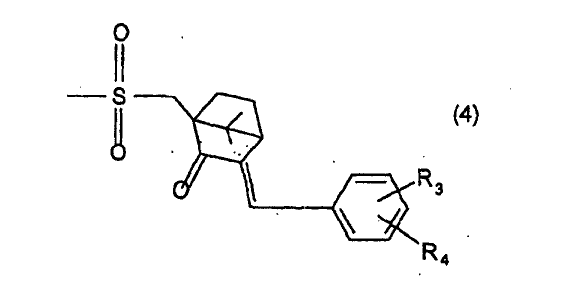 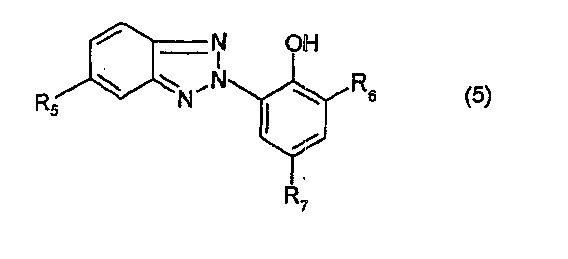 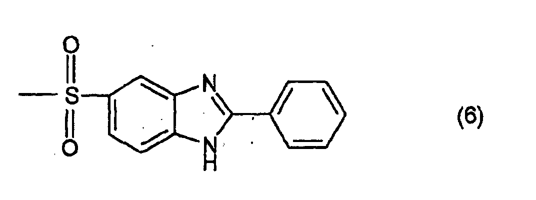 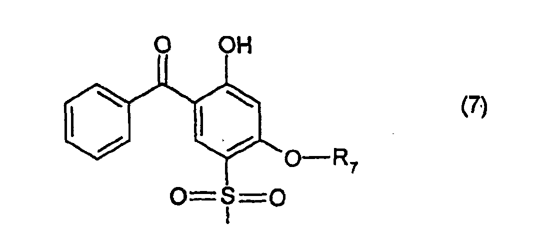
dans lesquelles :
- p est un entier de 1 à 3 ;
- R₂ désigne hydrogène, un radical alkyle en C₁-C₈, un radical O-alkyle en C₁-C₈, O-acyle en C₁-C₈ ;
- R₃ et R₄ similaires ou différents désignent H ou halogène ou alkyl ou alcoxy en C₁-C₈ ou encore un groupe de formule (8) suivant : 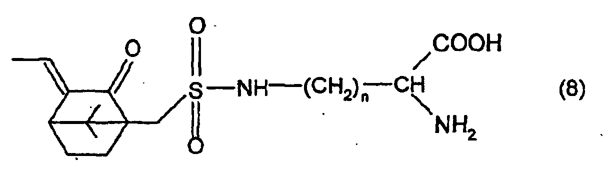
- R₅ représente un atome d'hydrogène ou d'halogène, un radical alkyle ou alcoxy en C₁-C₈ ou un radical divalent 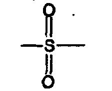
- R₆ représente un atome d'hydrogène, un radical alkyle en C₁-C₈ ou un radical divalent 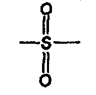 avec la restriction qu'au moins un des deux substituants R₅, R₆ représente 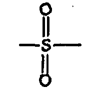
- R₇ représente un radical alkyle en C₁-C₈; ainsi que leurs sels d'acide ou de base dérivés.

Plus particulièrement, lorsque la matière sensible à protéger est la peau et/ou les cheveux, cette composition se présente sous la forme d'une composition cosmétique comprenant, dans un support cosmétiquement acceptable, une quantité efficace d'au moins un composé de formule (I).

Les composés de formule (I) conformes à l'invention peuvent se présenter sous forme de mélange d'isomères ou d'isomère pur (régio, énantio ou isomérie géométrie).

Parmi les composés de formule (I) préférentiels, on citera les dérivés d'ornithine (n = 3) et les dérivés de lysine (n= 4).

Parmi les composés de formule (I) conformes à l'invention, on peut citer plus particulièrement :
- la N^{ε}-cinnamoyl-L-lysine ;
- la N^{ε}-(4-méthoxycinnamoyl)-L-lysine ;
- la N^{ε}-[4-(4,7,7-triméthyl-3-oxo-bicyclo[2.2.1]hept-2-ylidèneméthyl)-benzènesulfonyl]-L-lysine.

La N^{ε}-cinnamoyl-L-lysine est connue de l'article « Syntheses of photosensitive Poly(amino acids) », NASAWA M. et KAMOGAWA H., Bulletin of the Chemical Society of Japan, vol 48 (9), pages 2588-2591 (1975). La 3-[(1-oxo-3-phényl-2-propényl)amino]-L-alanine et son monochlorhydrate sont connus de l'article « Structural determinants of inhibitory activity of N³-(4-methoxyfumaroyl)-L-2,3-Diaminopropanoic acid towards glucosamine-6-phosphate synthase », Andruszkiewicz R., Polish J. Chem., 67, pages 673-683 (1993).

L'invention a également pour objet les nouveaux composés d'aminoacides répondant à la formule (I) telle que définie précédemment à l'exclusion de la N^{ε}-cinnamoyl-L-lysine, et de la 3-[(1-oxo-3-phényl-2-propényl)amino]-L-alanine et son monochlorhydrate.

La présente invention a également pour objet un procédé de préparation des nouveaux composés de formule (I) définis ci-avant consistant :
- (a) dans une première étape à complexer la partie ₂HN-CH(COOH)- d'un monochlorhydrate d'aminoacide de formule (II) suivante : 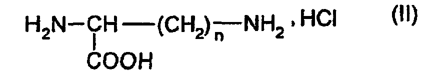 par un sulfate de cuivre dans un milieu basique (par exemple une solution aqueuse de soude) en présence d'un solvant organique polaire (tel que tétrahydrofurane, diméthylformamide ou acétone) ;
- (b) dans une deuxième étape, à faire réagir en présence d'un solvant organique polaire le complexe de cuivre ainsi obtenu avec un chlorure d'acide R₁COCl ou un chlorure de sulfonyle R₁SO₂Cl où R₁ a la même signification indiquée dans la formule (1) définie ci-dessus ;
- (c) dans une troisième étape, à récupérer par filtration le précipité obtenu dans l'étape (b) ;
- (d) à décomplexer le produit ainsi obtenu par un agent séquestrant (par exemple l'acide éthylène diaminotétraacétique).

Ces dérivés d'aminoacides sont insolubles ou substantiellement insolubles dans l'eau et dans les solvants organiques couramment utilisés en cosmétique notamment les huiles.

Par composés insolubles ou substantiellement insolubles, on entendra, au sens de la présente invention, des composés dont la solublilité dans l'eau est inférieure à 2 % en poids, dont la solubilité dans l'huile de vaseline est inférieure à 3% en poids, et enfin, dont la solubilité dans un mélange d'esters de triglycérides tel que le « Miglyol 812 » commercialisé par la société Dynamit Nobel est inférieure à 5%, également en poids.

Les dérivés d'aminoacides conformes à la présente invention peuvent être amenés sous une forme particulaire convenable par tout moyen ad-hoc tel que notamment broyage à sec ou en milieu solvant, tamisage, atomisation, micronisation, pulvérisation. La taille moyenne des particules sera en général inférieur à 50µm et variera plus préférentiellement de 0,01 µm à environ 20µm.

Les dérivés d'aminoacides conformes à la présente invention peuvent être utilisés comme filtres UV pour la protection solaire de la peau humaine et des cheveux. Ils peuvent également être utilisés comme agents photoprotecteurs de la lumière dans l'industrie des plastiques, des textiles, du verre (emballage, verres optiques, notamment pour lunetterie) et autres matériaux industriels comme les produits alimentaires.

Le ou les composés de formule (I) peuvent être présents dans la composition cosmétique selon l'invention dans des proportions comprises entre 0,1 et 20% en poids, par rapport au poids total de la composition, de préférence entre 0,1 et 15%.

La composition cosmétique de l'invention peut être utilisée comme composition protectrice de l'épidémie humain ou des cheveux ou comme composition antisolaire.

Les compositions cosmétiques antisolaires selon l'invention peuvent bien entendu contenir un ou plusieurs filtres organiques solaires complémentaires actifs dans l'UVA et/ou l'UVB (absorbeurs), hydrophiles ou lipophiles, autres bien sûr que les deux filtres mentionnés ci-avant. Ces filtres organiques complémentaires peuvent être notamment choisis parmi les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone ; les dérivés de β, β'-diphénylacrylate, les dérivés de benzimidazole ; les dérivés bis-benzoazolyle tels que ceux décrits dans les brevets EP-A-0669323 et US 2,463,264 ; les dérivés de bis-hydroxyphénolbenzotriazole tels que ceux décrits dans les demandes US 5237071, US 5166355, GB-A-2303549, DE 19726184 et EP-A-893119; les dérivés de l'acide p-aminobenzoïque ; les polymères hydrocarbonés filtres et les silicones filtres tels que ceux décrits notamment dans la demande WO-93/04665.

Comme exemples de filtres solaires actifs dans l'UV-A et/ou l'UV-B, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine;
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-bornan-2-on-méthylsulfate,
le 2-hydroxy-4-méthoxybenzophénone,
le 2-hydroxy-4-méthoxybenzophénone-5-sulfonate,
le 2,4-dihydroxybenzophénone,
la 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide α-(2-oxoborn-3-ylidène)-tolyl-4-sulfonique et ses sels solubles
la 3-(4'-sulfo)benzylidèn-bornan-2-one et ses sels solubles,
le 3-(4'méthylbenzylidène)-d,l-camphre,
le 3-benzylidène-d,l-camphre,
l'acide benzène 1,4-di(3-méthylidène-10-camphosulfonique) et ses sels solubles,
l'acide urocanique,
la 2,4,6-tris-[p-(2'-éthylhexyl-1'-oxycarbonyl)anilino]-1,3,5-triazine,
la 2-[p-(tertiobutylamido)anilino]-4,6-bis-[(p-(2'-éthylhexyl-1'-oxycarbonyl)anilino]-1,3,5-triazine,
la 2,4-bis {[4-2-éthyl-hexyloxy)]-2-hydroxy]-phenyl}-6-(4-méthoxy-phenyl)-1,3,5-triazine ;
le polymère de N-(2 et 4)-[(2-oxoborn-3-ylidèn)méthyl] benzyl]-acrylamide,
l'acide 1,4-bis-benzimidazolyl-phénylen-3,3',5,5'-tétrasulfonique et ses sels solubles.
la 2,2'-méthylène-bis-[6-(2H-benzotriazol-2-yl)-4-(1,1,3,3-tétraméthylbutyl)phénol],
le composé (2,2'-méthylène-bis-[6-(2H-benzotriazol-2-yl)-4-(methyl)phénol],
les polyorganosiloxanes à fonction benzalmalonate
les polyorganosiloxanes à fonction benzotriazole tel que le Drométrizole Trisiloxane.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium ou leurs mélanges qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les propulseurs, les agents alcalinisants ou acidifiants, les polymères, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les homopolymères d'acide acrylique réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, attachées intrinsèquement aux dérivés d'aminoacides conformes à l'invention ne soient pas, ou substantiellement pas, altérées par la ou les adjonctions envisagées.

Les compositions de rinvention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. *J. Mol. Biol*. **1965,** 13, 238, FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

La présente invention a également pour objet l'utilisation d'au moins un composé de formule (I) pour la fabrication de compositions destinées à protéger des matériaux Industriels sensibles au rayonnement ultraviolet, en particulier au rayonnement solaire.

La présente invention a encore pour objet l'utilisation d'au moins un composé de formule (I) pour la fabrication de compositions cosmétiques pour la protection de la peau et/ou des cheveux contre le rayonnement ultraviolet, en particulier le rayonnement solaire.

Ces matériaux industriels photosensibles peuvent être notamment des verres organiques et/ou minéraux, des matières plastiques, des textiles, des peintures, des vernis, des produits alimentaires, etc....

Les composés de l'invention peuvent être appliquées en quantité efficace sur la surface desdits matériaux industriels photosensibles ou bien être incorporés directement dans la composition desdits matériaux industriels.

La présente invention a donc également pour objet un procédé de protection d'un matériau industriel photosensible, contre les effets néfastes des rayonnements UV, en particulier du rayonnement solaire consistant à incorporer dans ledit matériau une quantité efficace d'un composé de formule (I).

Dans une autre forme de réalisation de l'invention, le procédé de protection peut consister à appliquer sur la surface dudit matériau industriel photosensible une quantité efficace d'un composé de formule (I).

Des exemples concrets, mais nullement limitatifs, illustrant l'invention, vont maintenant être donnés.

### EXEMPLE 1 : Préparation de la N ^{ε}-cinnamoyl-L-Lysine (isomérie géométrique trans)

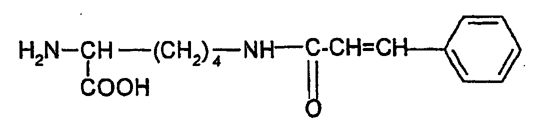

### Mode opératoire

Dans un tricol de 250 ml muni d'un thermomètre et d'une ampoule à introduction de 50 ml , 10 g de L-lysine monochlorhydrate (54,75 mmoles) sont dissous dans 44 ml de solution de soude à 10% (2 équivalents). Une solution de 6,8 g (0,5 équivalent) de sulfate de cuivre pentahydrate dans 40 ml d'eau est introduite dans le milieu réactionnel suivi de 4,6 g d'hydrogénocarbonate de sodium et 20 ml de solvont organique (tétrahydrofurane, acétone ou diméthylformamide). Une solution contenant 1 équivalent de chlorure d'acide cinnamique dans 20 ml de solvant est préparée puis versée goutte à goutte sur le mélange réactionnel homogène refroidi à la température de 5 °C. Après une nuit sous agitation à la température ambiante, on filtre le milieu réactionnel et on récupère le précipité bleu correspondant au produit final sous forme de complexe de cuivre . Ledit précipité est lavé à l'eau et à l'acétone, puis séché à l'étuve sous vide. Pour éliminer le cuivre, on traite x moles de complexe au reflux pendant 4 heures par 3x moles de sel disodique dihydraté de l'acide éthylène diaminotétraacétique en solution à 10 %. Le traitement peut être répété plusieurs fois jusqu'à obtention du produit décomplexé. On obtient 8,9 g de produit final soit un rendement de 60%

### Analyses

Point de fusion (Banc KOFLER) : >260°C
Analyse élémentaire (C₁₆H₂₀N₂O₃) ; Poids moléculaire : 276,34

| | C | H | N | O | Cu |
|---|---|---|---|---|---|
| % théorique : | 65,2 | 7,3 | 10,14 | 17,37 | |
| % trouvé | 65,04 | 7,36 | 9,95 | 17,35 | 698 ppm |

Granulométrie en nombres (moyenne) mesurée au Coulter Counter TA2 :
1,73 µm (écart type : 0,81 µm)

Pouvoir rotatoire déterminé par polarimètre PERKIN-ELMER modèle 241 :
[ α ] _{D} = + 17 ° (c=2 ; HCl 6N ; 20°C)

Les spectres de masse et de RMN ¹H sont conformes à la structure attendue.

### Propriétés d'absorption UV/diffusion de la lumière :

On mesure le spectre d'absorption UV du composé en dispersion à 5% dans l'huile de vaseline . La transmission et la réflexion totale de la dispersion est déterminée sur un film de 60 µm avec un spectrophotomètre muni d'une sphère d'intégration.

On observe un spectre massif entre 310 et 350 nm et présentant :
- un maximum d'absorption à 260 nm et un épaulement à 305 nm
- une densité optique à 310 nm de 0,8.

### EXEMPLE 2 : Préparation de la N ^{ε}-(4-méthoxycinnamoyl)-L-Lysine (isomérie géométrique trans)

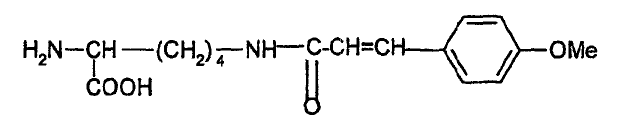

### Mode opératoire

On opère dans les mêmes conditions que l'exemple 1 en utilisant comme chlorure d'acide, le chlorure de 4-méthoxycinnamoyle. On obtient 6,3 g de produit final soit un rendement de 38%.

### Analyses

Point de fusion (Banc KOFLER) : >260°C
Analyse élémentaire (C₁₆H₂₂N₂O₄) ; Poids moléculaire : 306,37

| | C | H | N | O |
|---|---|---|---|---|
| % théorique : | 62,73 | 7,24 | 9,14 | 20,89 |
| % trouvé | 62,50 | 7,22 | 9,19 | 20,88 |

Granulométrie en nombres (moyenne) mesurée au Coulter Counter TA2 :
10,72 µm (écart type : 5,63 pm)

Pouvoir rotatoire déterminé par polarimètre PERKIN-ELMER modèle 241 :
[ α ] _{D} = + 17,6 ° (c=2, HCl 6N ; 20°C)

Les spectres de masse et de RMN ¹H sont conformes à la structure attendue.

### Propriétés d'absorption UV/diffusion de la lumière :

On mesure le spectre d'absorption UV du composé en dispersion à 5% dans l'huile de vaseline . La transmission et la réflexion totale de la dispersion est déterminée sur un film de 60 µm avec un spectrophotomètre muni d'une sphère d'intégration (8 essais).

On observe un spectre présentant un plateau entre 310 et 250 nm et ayant :
- un maximum d'absorption à 300 nm et un épaulement à 320 nm
- une densité optique à 310 nm de 0,4.

### EXEMPLE 3 : Préparation de la N^{ε}-[4-(4,7,7-triméthyl-3-oxo-bicyclo[2.2.1]hept-2-ylidèneméthyl)-benzènesulfonyl]-L-lysine.

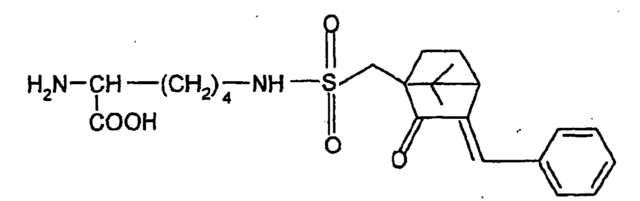

### Mode opératoire

On opère dans les mêmes conditions que l'exemple 1 en utilisant comme chlorure de sulfonyle, le chlorure de 4-(4,7,7-triméthyl-3-oxo-bicyclo[2.2.1]hept-2-ylidèneméthyl)-benzènesulfonyle. On obtient 16 g de produit final soit un rendement de 66%.

### Analyses

Point de fusion (Banc KOFLER) : 246-248°C
Analyse élémentaire (C₂₃H₃₂N₂O₆S) ; Poids moléculaire : 448,58

| | C | H | N | O | S |
|---|---|---|---|---|---|
| % théorique : | 61,58 | 7,19 | 6,24 | 17,83 | 7,15 |
| % trouvé | 61,17 | 7,24 | 6,17 | 18,29 | 7,14 |

Granulométrie en nombres (moyenne) mesurée au Coulter Counter TA2 :
1,95 µm (écart type : 1,05 µm)

Pouvoir rotatoire déterminé par polarimètre PERKIN-ELMER modèle 241 :
[ α ] _{D} = + 15,5 ° (c=2, CH₃COOH, 20°C)

Les spectres de masse et de RMN ¹H sont conformes à la structure attendue.

### Propriétés d'absorption UV/diffusion de la lumière :

On mesure le spectre d'absorption UV du composé en dispersion à 5% dans l'huile de vaseline . La transmission et la réflexion totale de la dispersion est déterminée sur un film de 60 µm avec un spectrophotomètre muni d'une sphère d'intégration (8 essais).

On observe un spectre présentant une bande résolue et ayant :
- un maximum d'absorption à 296 nm , un premier épaulement à 320 nm et un deuxième épaulement à 282 ;
- une densité optique à 310 nm de 1,2.

### EXEMPLE DE FORMULATION

| **COMPOSITION (émulsion H/E)** | **% en polds** |
|---|---|
| Mélange mono /distearate de glycerol / stéarate de polyéthylène | 2 |
| glycol (100 OE) | |
| (ARLACEL 165 FL - UNIQUEMA) | |
| Alcool stearylique | 1 |
| (LANETTE 18 - HENKEL) | |
| Acide stéarique d'huile de palme | 2 |
| (STEARINE TP - STEARINERIE DUBOIS) | |
| Poly dimethylsiloxane | 1 |
| (DOW CORNING 200 FLUID - DOW CORNING) | |
| Petrolatum | 3 |
| (VASELINE BLANCHE ) | |
| Huile de vaseline | 15 |
| (MARCOL 82- ESSO) | |
| Acide polyacrylique | 0.3 |
| (CARBOPOL 980-GOODRICH) | |
| 4-(4,7,7-triméthyl-3-oxo-bicyclo[2.2.1]hept-2-ylidèneméthyl)-benzènesulfonyle (exemple 3) | 10 |
| 1,3-butylène glycol | 10 |
| Triethanolamine | 0.4 |
| Conservateurs | qs |
| Eau démineralisée | qsp 100 g |

## Revendications

1. Dérivé d'aminoacide répondant à la formule suivante (I) : 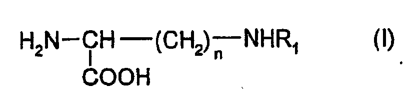 dans laquelle :
- n est un nombre entier de 1 à 4
- le radical R₁ désigne l'un des groupes de formule (1), (2) ou (3) (4), (5) (6) ou (7) suivants : 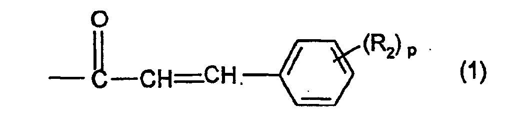 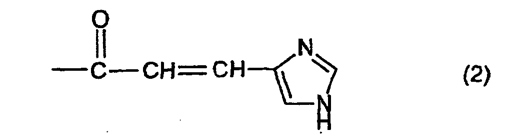 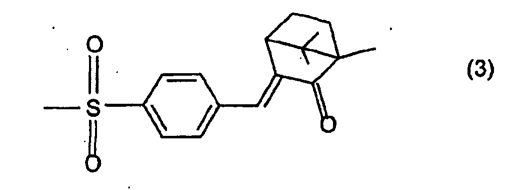 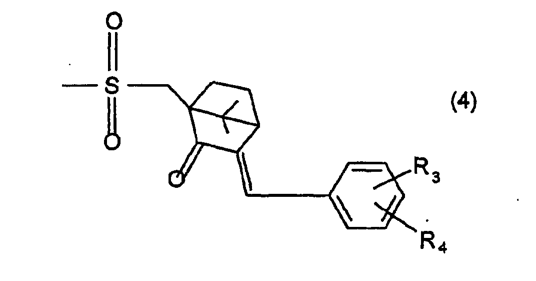 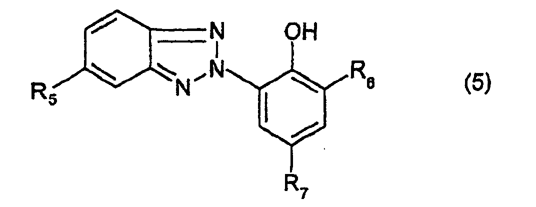 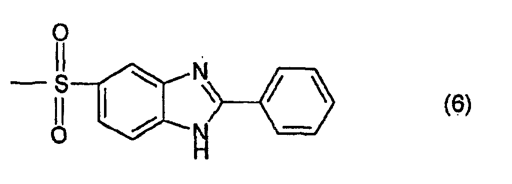 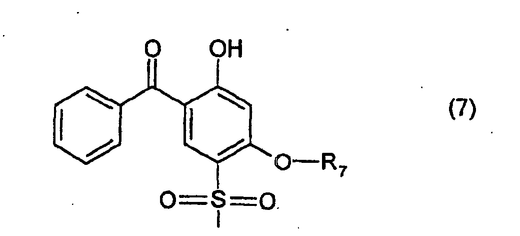
dans lesquelles :
- p est un entier de 1 à 3 ;
- R₂ désigne hydrogène, un radical alkyle en C₁-C₈, un radical O-alkyle en C₁-C₈, O-acyle en C₁-C₈ ;
- R₃ et R₄ similaires ou différents désignent H ou halogène ou alkyl ou alcoxy en C₁-C₈ ou encore un groupe de formule (8) suivant : 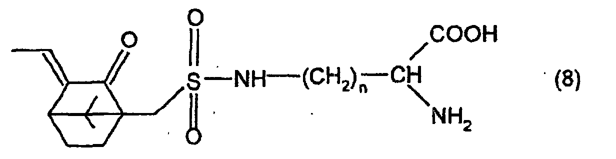
- R₅ représente un atome d'hydrogène ou d'halogène, un radical alkyle ou alcoxy en C₁-C₈ ou un radical divalent 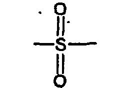
- R₆ représente un atome d'hydrogène, un radical alkyle en C₁-C₈ ou un radical divalent 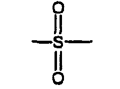 avec la restriction qu'au moins un des deux substituants R₅, R₆ représente 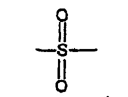
- R₇ représente un radical alkyle en C₁-C₈ ; ainsi que ses sels d'acide ou de base dérivés ; ledit composé se présente sous forme de mélange d'isomères ou d'isomère pur,
à l'exclusion de la N^{ε}-cinnamoyl-L-lysine, et de la 3-[(1-oxo-3-phényl-2-propényl)amino]-L-alanine et son monochlorhydrate.

2. Dérivé d'aminoacide selon la revendication 1, **caractérisés par le fait qu'**ils est choisi parmi les dérivés d'ornithine (n = 3) et les dérivés de lysine (n= 4).

3. Dérivé d'aminoacide selon la revendication 1 ou 2, **caractérisé par le fait qu'**il est choisi parmi :
- la N^{ε}-(4-méthoxycinnamoyl)-L-lysine ;
- la N^{ε}-[4-(4,7,7-triméthyl-3-oxo-bicyclo[2.2.1]hept-2-ylidèneméthyl)-benzènesulfonyl]-L-lysine.

4. Dérivé d'aminoacide selon l'une quelconque des revendications 1 à 3, **caractérisé par le fait qu'**ils se présentent sous forme de particules ayant une taille moyenne inférieure à 50µm.

5. Dérivé d'aminoacide selon l'une quelconque des revendications 1 à 4, **caractérisé par le fait qu'**ils se présentent sous forme de particules ayant une taille moyenne allant de 0,01µm à environ 20µm.

6. Procédé de préparation des composés de formule (I) tels que définis dans l'une quelconque des revendications 1 à 5 :
- (a) dans une première étape à complexer la partie ₂HN-CH(COOH)- d'un monochlorhydrate d'aminoacide de formule (II) suivante : 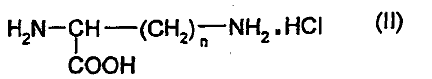 par un sulfate de cuivre dans un milieu basique en présence d'un solvant polaire ;
- (b) dans une deuxième étape, à faire réagir en présence d'un solvant organique polaire le complexe de cuivre ainsi obtenu avec un chlorure d'acide R₁COCl ou un chlorure de sulfonyle R₁SO₂Cl où R₁ a la même signification indiquée dans la formule (I) définie dans la revendication 1 ;
- (c) dans une troisième étape, à récupérer par filtration le précipité obtenu dans l'étape (b) ;
- (d) à décomplexer le produit ainsi obtenu par un agent séquestrant.

7. Composition destinée à protéger une matière sensible au rayonnement ultraviolet, en particulier au rayonnement solaire, **caractérisée par le fait qu'**elle comprend une quantité efficace d'au moins un composé de formule (1) suivante : 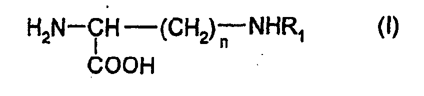 dans laquelle :
- n est un nombre entier de 1 à 4
- le radical R₁ désigne l'un des groupes de formule (1), (2) ou (3) (4), (5) (6) ou (7) suivants : 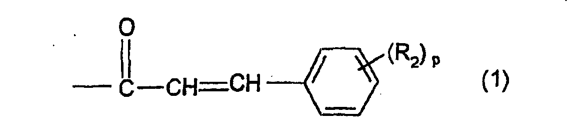 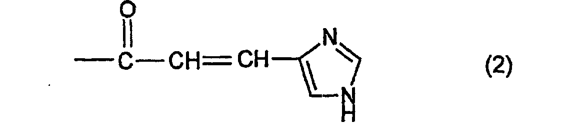 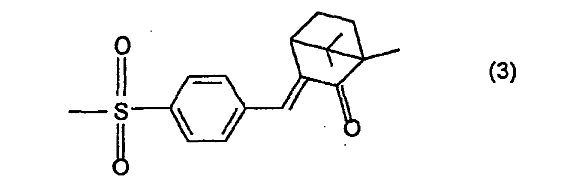 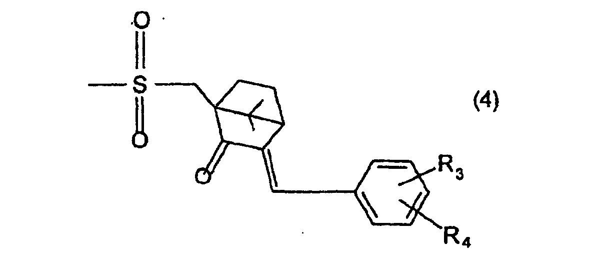 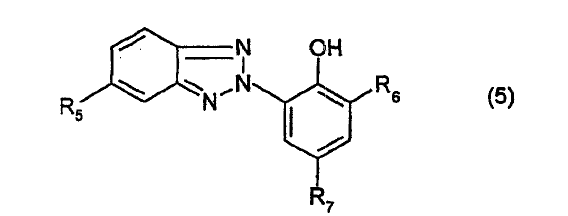 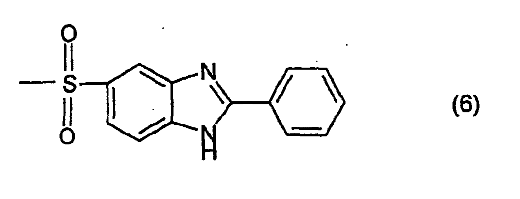 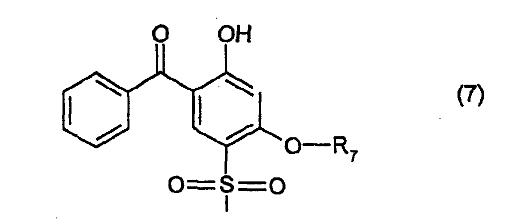
dans lesquelles :
- p est un entier de 1 à 3 ;
- R₂ désigne hydrogène, un radical alkyle en C₁-C₈, un radical O-alkyle en C₁-C₈, O-acyle , en C₁-C₈ ;
- R₃ et R₄ similaires ou différents désignent H ou halogène ou alkyl ou alcoxy en C₁-C₈ ou encore un groupe de formule (8) suivant : 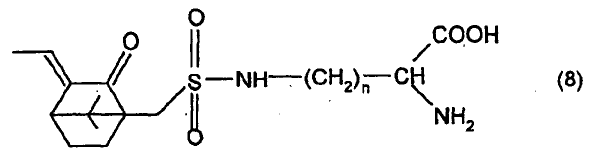
- R₆ représente un atome d'hydrogène ou d'halogène, un radical alkyle ou alcoxy en C₁-C₈ ou un radical divalent 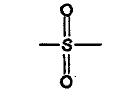
- R₆ représente un atome d'hydrogène, un radical alkyle en C₁-C₈ ou un radical divalent 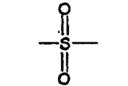 avec la restriction qu'au moins un des deux substituants R₅, R₆ représente 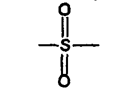
- R₇ représente un radical alkyle en C₁-C₈ ; ainsi que ses sels d'acide ou de base dérivés ; ledit composé se présente sous forme de mélange d'isomères ou d'isomère pur.

8. Composition cosmétique photoprotectrice de la peau et/ou les cheveux, **caractérisée par le fait qu'**elle comprend dans un support cosmétiquement acceptable une quantité efficace d'au moins un composé de formule (I) suivante : 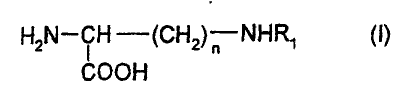 dans laquelle :
- n est un nombre entier de 1 à 4
- le radical R₁ désigne l'un des groupes de formule (1), (2) ou (3) (4), (5) (6) ou (7) suivants : 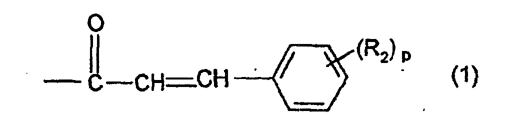 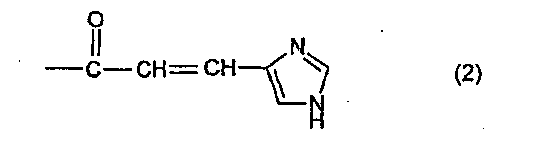 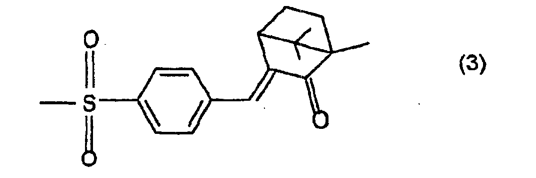 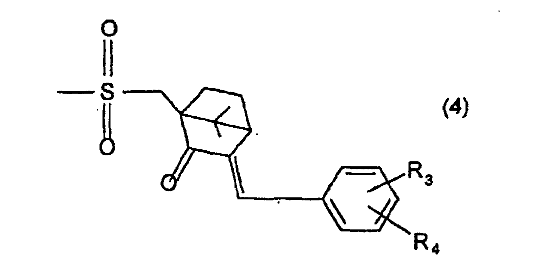 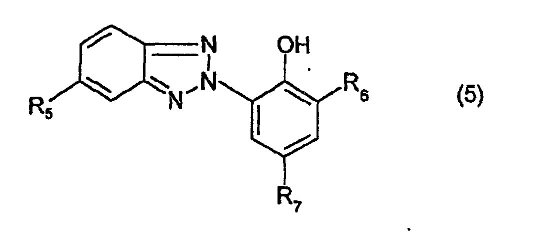 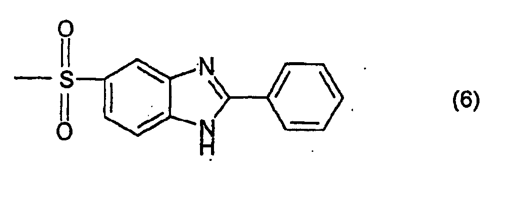 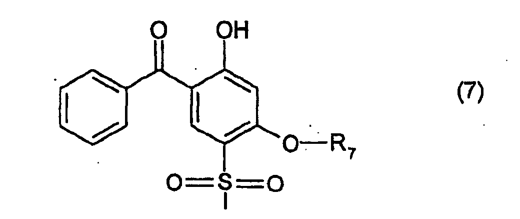
dans lesquelles :
- p est un entier de 1 à 3 ;
- R₂ désigne hydrogène, un radical alkyle en C₁-C₈, un radical O-alkyle en C₁-C₈, O-acyle en C₁-C₈ ;
- R₃ et R₄ similaires ou différents désignent H ou halogène ou alkyl ou alcoxy en C₁-C₈ ou encore un groupe de formule (8) suivant : 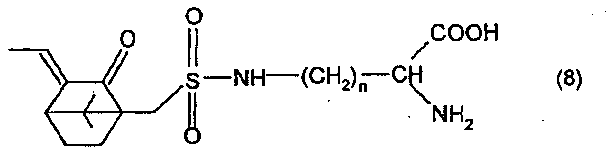
- R₅ représente un atome d'hydrogène ou d'halogène, un radical alkyle ou alcoxy en C₁-C₈ ou un radical divalent 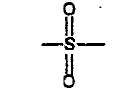
- R₆ représente un atome d'hydrogène, un radical alkyle en C₁-C₈ ou un radical divalent 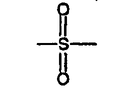 avec la restriction qu'au moins un des deux substituants R₅, R₆ représente 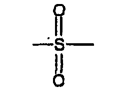
- R₇ représente un radical alkyle en C₁-C₈ ; ainsi que ses sels d'acide ou de base dérivés ; ledit composé se présente sous forme de mélange d'isomères ou d'isomère pur.

9. Composition selon la revendication 7 ou 8, **caractérisée par le fait que** le composé de formule (I) est choisi parmi les dérivés d'ornithine (n = 3) et les dérivés de lysine (n= 4).

10. Composition selon l'une des revendications 7 à 9, **caractérisée par le fait que** le composé de formule (1) est choisi parmi :
- la N^{ε}-cinnamoyl-L-lysine ;
- la N^{ε}-(4-méthoxycinnamoyl)-L-lysine ;
- la N^{ε}-[4-(4,7,7-triméthyl-3-oxo-bicyclo[2.2.1]hept-2-ylidèneméthyl)-benzènesulfonyl]-L-lysine.

11. Composition selon l'une quelconque des revendications 7 à 10, **caractérisée par le fait que** le composé de formule (I) se présente sous forme de particules ayant une taille moyenne inférieure à 50µm.

12. Composition selon l'une quelconque des revendications 7 à 11, **caractérisée par** le fait le composé de formule (I) se présente sous forme de particules ayant une taille moyenne allant de 0,01µm à environ 20µm.

13. Composition selon l'une quelconque des revendications 8 à 12, **caractérisée par le fait qu'**elle contient de 0,1 à 20 % en poids, par rapport au poids total de la composition, de composé de formule (I).

14. Composition selon l'une quelconque des revendications 8 à 13, **caractérisée par le fait que** ledit support cosmétiquement acceptable se présente sous la forme d'une émulsion de type huile-dans-eau.

15. Composition selon l'une quelconque des revendications 8 à 14, **caractérisée par le fait qu'**elle comprend en outre un ou plusieurs filtres organiques complémentaires actifs dans l'UV-A et/ou UV-B, hydrophiles ou lipophiles.

16. Composition selon la revendication 15, **caractérisée par** le falt que lesdits filtres organiques complémentaires sont choisis parmi les dérivés cinnamiques ; les dérivés de dibenzoylméthane ; les dérivés salicyliques, les dérivés du camphre ; les dérivés de triazine ; les dérivés de la benzophénone ; les dérivés de β,β'-diphénylacrylate ; les dérivés de benzimidazole ; les dérivés bis-benzoazolyle ; les dérivés de bis-hydroxyphénolbenzotriazole ; les dérivés de l'acide p-aminobenzoïque ; les polymères hydrocarbonés filtres et les silicones filtres.

17. Composition selon l'une quelconque des revendications 8 à 16, **caractérisée par le fait qu'**elle comprend en outre, à titre d'agents photoprotecteurs UV complémentaires, des pigments ou des nanopigments d'oxydes métalliques, enrobés ou non.

18. Composition selon la revendication 17, **caractérisée par le fait que** lesdits pigments ou nanopigments sont choisis parmi les oxydes de titane, de zinc, de fer, de zirconium, de cérium et leurs mélanges, enrobés ou non.

19. Composition selon l'une quelconque des revendications 8 à 18, **caractérisée par le fait qu'**elle comprend en outre au moins un agent de bronzage et/ou de brunissage artificiel de la peau.

20. Composition selon l'une quelconque des revendications 8 à 19, **caractérisée par le fait qu'**elle comprend en outre au moins un adjuvant choisi parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

21. Composition selon l'une quelconque des revendications 8 à 20, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un stick, d'un bâtonnet solide, d'une mousse ou d'un spray.

22. Composition selon l'une quelconque des revendications 8 à 21, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

23. Composition selon l'une quelconque des revendications 8 à 22, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

24. Utilisation d'au moins un composé de formule (I) tel que défini dans les revendications 8 à 12 pour la fabrication de compositions destinées à protéger des matériaux industriels sensibles au rayonnement ultraviolet, en particulier au rayonnement solaire.

25. Utilisation selon la revendication 24 où les matériaux industriels photosensibles sont des verres organiques et/ou minéraux, des matières plastiques, des textiles, des peintures, des vernis, des produits alimentaires.

26. Utilisation d'au moins un composé de formule (I) tel que défini dans l'une quelconque des revendications 8 à 12 pour la fabrication de compositions cosmétiques pour la protection de la peau et/ou des cheveux contre le rayonnement ultraviolet, en particulier le rayonnement solaire.

27. Procédé de protection d'un matériau industriel photosensible, contre les effets néfastes des rayonnements UV, en particulier du rayonnement solaire consistant à incorporer dans la composition dudit matériau une quantité efficace d'un composé de formule (I) tel que défini dans les revendications 8 à 12.

28. Procédé de protection d'un matériau industriel photosensible, contre les effets néfastes des rayonnements UV, en particulier du rayonnement solaire consistant à appliquer sur la surface dudit matériau une quantité efficace d'un composé de formule (I) tel que défini dans les revendications 8 à 12.

## Claims

1. Amino acid derivative corresponding to the following formula (I): 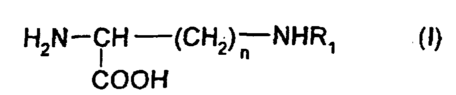 in which:
- n is an integer from 1 to 4;
- the radical R₁ denotes one of the following groups of formula (1), (2), (3), (4), (5), (6) or (7): 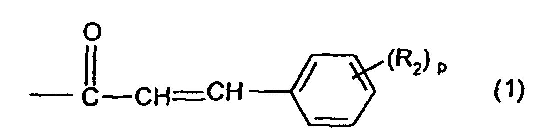 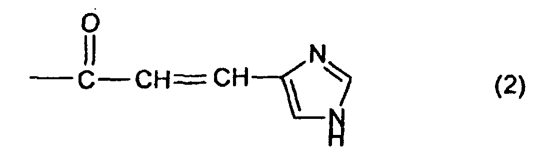 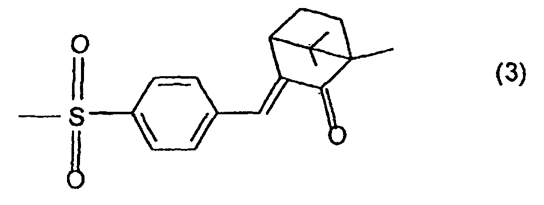 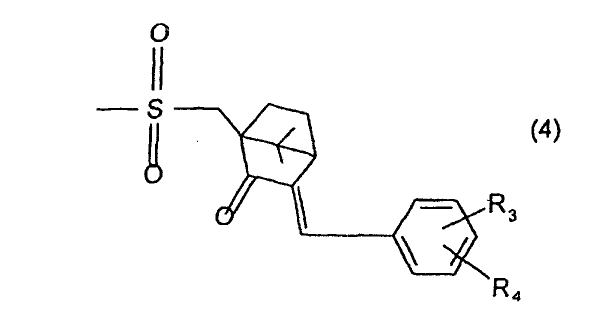 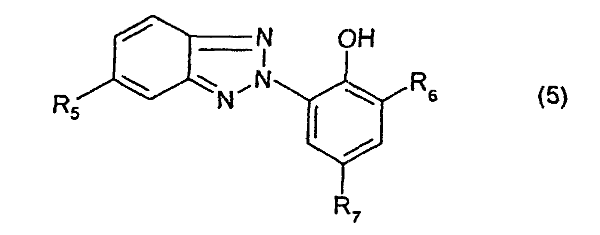 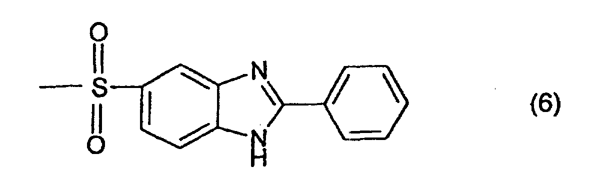 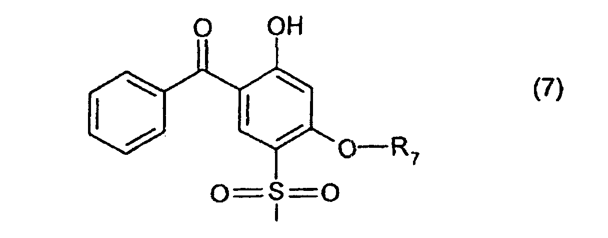
in which:
- p is an integer from 1 to 3;
- R₂ denotes hydrogen, a C₁-C₈ alkyl radical, a C₁-C₈ 0-alkyl radical or a C₁-C₈ O-acyl radical;
- R₃ and R₄, which are alike or different, denote H or halogen or C₁-C₈ alkyl or alkoxy or a following group of formula (8): 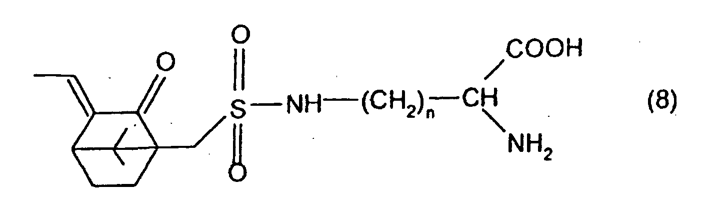
- R₅ represents a hydrogen or halogen atom, a C₁-C₈ alkyl or alkoxy radical, or a divalent radical 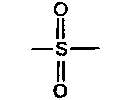
- R₆ represents a hydrogen atom, a C₁-C₈ alkyl radical or a divalent radical 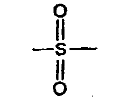 with the restriction that at least one of the two substituents R₅ and R₆ represents 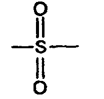
- R₇ represents a C₁-C₈ alkyl radical; and its derived acid or base salts;
the said compound being provided in the form of a mixture of isomers or of a pure isomer, with the exception of N^{ε}-cinnamoyl-L-lysine and of 3-[(1-oxo-3-phenyl-2-propenyl)amino]-L-alanine and its monohydrochloride.

2. Amino acid derivative according to Claim 1, **characterized in that** it is chosen from ornithine (n = 3) derivatives and lysine (n = 4) derivatives.

3. Amino acid derivative according to Claim 1 or 2, **characterized in that** it is chosen from:
- N^{ε}-(4-methoxycinnamoyl)-L-lysine;
- N^{ε}-[4-(4,7,7-trimethyl-3-oxobicyclo[2.2.1]hept-2-ylidenemethyl)benzenesulphonyl]-L-lysine.

4. Amino acid derivative according to any one of Claims 1 to 3, **characterized in that** it is provided in the form of particles having a mean size of less than 50 µm.

5. Amino acid derivative according to any one of Claims 1 to 4, **characterized in that** it is provided in the form of particles having a mean size ranging from 0.01 µm to approximately 20 µm.

6. Process for the preparation of the compounds of formula (I) as defined in any one of Claims 1 to 5:
- (a), in a first stage, in complexing the NH₂-CH(COOH)-part of an amino acid monohydrochloride of following formula (II): 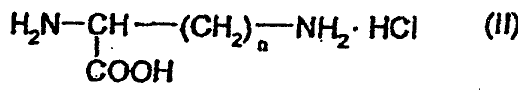 with a copper sulphate in a basic medium in the presence of a polar solvent;
- (b), in a second stage, in reacting, in the presence of a polar organic solvent, the copper complex thus obtained with an acid chloride R₁COCl or a sulphonyl chloride R₁SO₂Cl, where R₁ has the same meaning indicated in the formula (I) defined in Claim 1;
- (c), in a third stage, in recovering, by filtration, the precipitate obtained in stage (b);
- (d) in decomplexing the product thus obtained with a sequestering agent.

7. Composition intended to protect a material sensitive to ultraviolet radiation, in particular to solar radiation, **characterized in that** it comprises an effective amount of at least one compound of following formula (I): 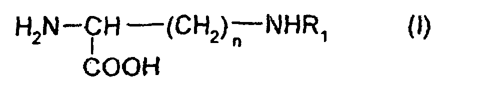 in which:
- n is an integer from 1 to 4;
- the radical R₁ denotes one of the following groups of formula (1), (2) , (3), (4), (5), (6) or (7) : 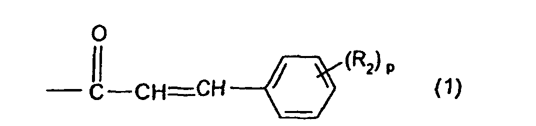 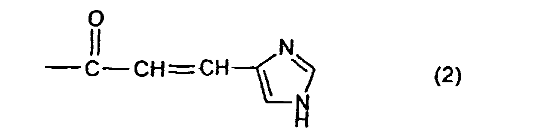 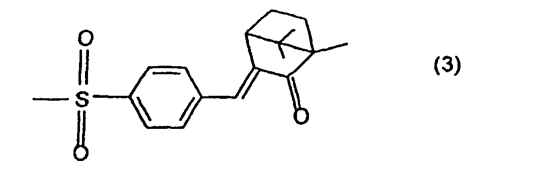 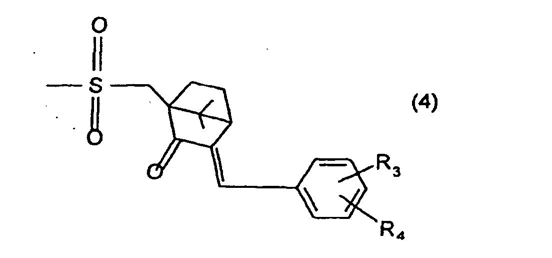 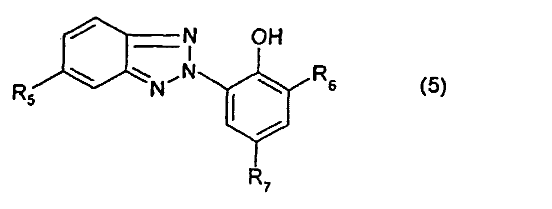 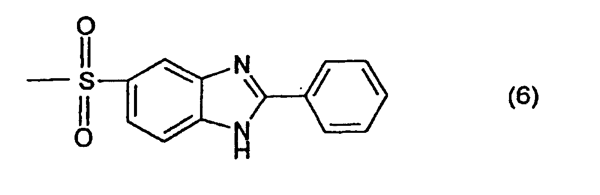 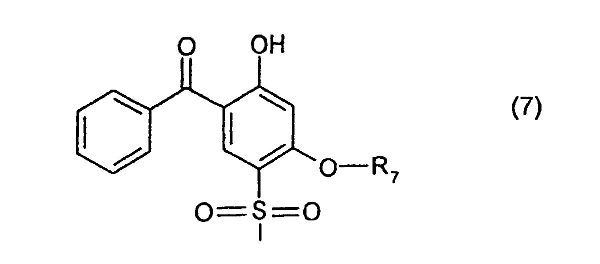
in which:
- p is an integer from 1 to 3;
- R₂ denotes hydrogen, a C₁-C₈ alkyl radical, a C₁-C₈ O-alkyl radical or a C₁-C₈ O-acyl radical;
- R₃ and R₄, which are alike or different, denote H or halogen or C₁-C₈ alkyl or alkoxy or a following group of formula (8) : 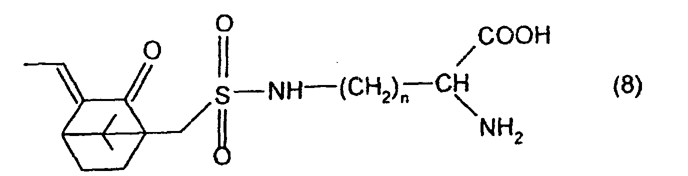
- R₅ represents a hydrogen or halogen atom, a C₁-C₈ alkyl or alkoxy radical, or a divalent radical 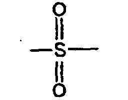
- R₆ represents a hydrogen atom, a C₁-C₈ alkyl radical or a divalent radical 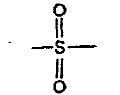 with the restriction that at least one of the two substituents R₅ and R₆ represents 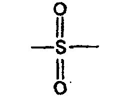
- R₇ represents a C₁-C₈ alkyl radical; and its derived acid or base salts;
the said compound being provided in the form of a mixture of isomers or of a pure isomer.

8. Photoprotective cosmetic composition for the skin and/or the hair, **characterized in that** it comprises, in a cosmetically acceptable vehicle, an effective amount of at least one compound of following formula (I): 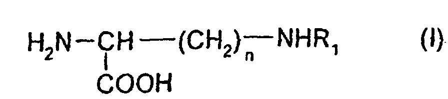 in which:
- n is an integer from 1 to 4;
- the radical R₁ denotes one of the following groups of formula (1), (2), (3), (4), (5), (6) or (7) : 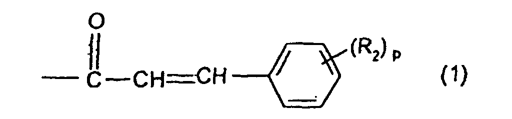 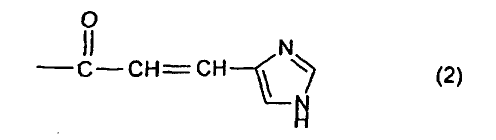 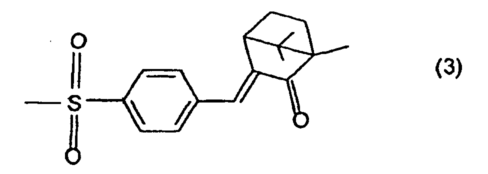 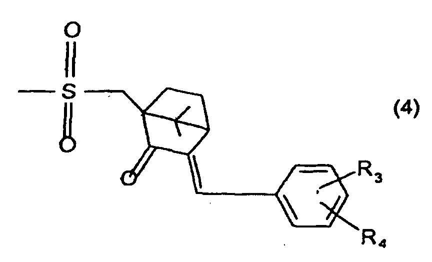 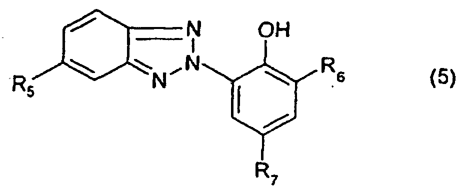 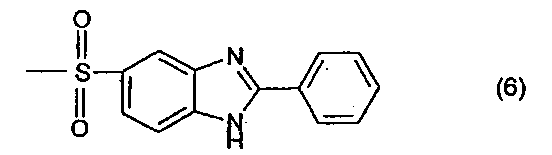 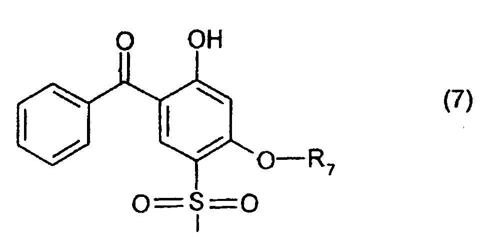
in which:
- p is an integer from 1 to 3;
- R₂ denotes hydrogen, a C₁-C₈ alkyl radical, a C₁-C₈ O-alkyl radical or a C₁-C₈ O-acyl radical;
- R₃ and R₄, which are alike or different, denote H or halogen or C₁-C₈ alkyl or alkoxy or a following group of formula (8): 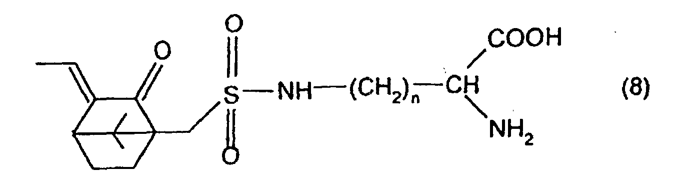
- R₅ represents a hydrogen or halogen atom, a C₁-C₈ alkyl or alkoxy radical, or a divalent radical 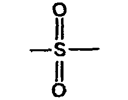
- R₆ represents a hydrogen atom, a C₁-C₈ alkyl radical or a divalent radical 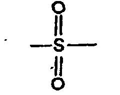 with the restriction that at least one of the two substituents R₅ and R₆ represents 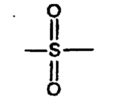
- R₇ represents a C₁-C₈ alkyl radical; and its derived acid or base salts;
the said compound being provided in the form of a mixture of isomers or of a pure isomer.

9. Composition according to Claim 7 or 8, **characterized in that** the compound of formula (I) is chosen from ornithine (n = 3) derivatives and lysine (n = 4) derivatives.

10. Composition according to one of Claims 7 to 9, **characterized in that** the compound of formula (I) is chosen from:
- N^{ε}-cinnamoyl-L-lysine;
- N^{ε}-(4-methoxycinnamoyl)-L-lysine;
- N^{ε}-[4-(4,7,7-trimethyl-3-oxobicyclo[2.2.2]hept-2-ylidenemethyl)benzenesulphonyl]-L-lysine.

11. Composition according to any one of Claims 7 to 10, **characterized in that** the compound of formula (I) is provided in the form of particles having a mean size of less than 50 µm.

12. Composition according to any one of Claims 7 to 11, **characterized in that** the compound of formula (I) is provided in the form of particles having a mean size ranging from 0.01 µm to approximately 20 µm.

13. Composition according to any one of Claims 8 to 12, **characterized in that** it comprises from 0.1 to 20% by weight, with respect to the total weight of the composition, of compound of formula (I).

14. Composition according to any one of Claims 8 to 13, **characterized in that** the said cosmetically acceptable vehicle is provided in the form of an emulsion of oil-in-water type.

15. Composition according to any one of Claims 8 to 14, **characterized in that** it furthermore comprises one or more additional, hydrophilic or lipophilic, organic screening agents which are active in the UV-A and/or UV-B regions.

16. Composition according to Claim 15, **characterized in that** the said additional organic screening agents are chosen from cinnamic derivatives, dibenzoylmethane derivatives, salicylic derivatives, camphor derivatives, triazine derivatives, benzophenone derivatives, β,β-diphenylacrylate derivatives, benzimidazole derivatives, bisbenzoazolyl derivatives, bishydroxyphenolbenzotriazole derivatives, p-aminobenzoic acid derivatives, or screening hydrocarbonaceous polymers and screening silicones.

17. Composition according to any one of Claims 8 to 16, **characterized in that** it furthermore comprises, as additional UV photoprotective agents, pigments or nanopigments formed of metal oxides which are coated or non-coated.

18. Composition according to Claim 17, **characterized in that** the said pigments or nanopigments are chosen from titanium, zinc, iron, zirconium or cerium oxides and their mixtures which are coated or non-coated.

19. Composition according to any one of Claims 8 to 18, **characterized in that** it additionally comprises at least one agent for the artificial tanning and/or browning of the skin.

20. Composition according to any one of Claims 8 to 19, **characterized in that** it additionally comprises at least one adjuvant chosen from fatty substances, organic solvents, thickeners, softeners, antioxidants, opacifiers, stabilizers, emollients, hydroxy acids, antifoaming agents, moisturizing agents, vitamins, fragrances, preservatives, surfactants, fillers, sequestering agents, polymers, propellants, basifying or acidifying agents, or dyes.

21. Composition according to any one of Claims 8 to 20, **characterized in that** it is a composition which protects the human epidermis or an anti-sun composition and that it is provided in the form of a nonionic vesicular dispersion, of an emulsion, in particular of an emulsion of oil-in-water type, of a cream, of a milk, of a gel, of a cream gel, of a suspension, of a dispersion, of a powder, of a solid stick, of a foam or of a spray.

22. Composition according to any one of Claims 8 to 21, **characterized in that** it is a composition for making up the eyelashes, eyebrows or skin and **in that** it is provided in the anhydrous or aqueous, pasty or solid form of an emulsion, of a suspension or of a dispersion.

23. Composition according to any one of Claims 8 to 22, **characterized in that** it is a composition intended for the protection of the hair against ultraviolet rays and **in that** it is provided in the form of a shampoo, of a lotion, of a gel, of an emulsion or of a nonionic vesicular dispersion.

24. Use of at least one compound of formula (I) as defined in Claims 8 to 12 in the manufacture of compositions intended to protect industrial materials which are sensitive to ultraviolet radiation, in particular to solar radiation.

25. Use according to Claim 24, where the photosensitive industrial materials are organic and/or inorganic glasses, plastics, textiles, paints, varnishes or foodstuffs.

26. Use of at least one compound of formula (I) as defined in any one of Claims 8 to 12 in the manufacture of cosmetic compositions for the protection of the skin and/or hair against ultraviolet radiation, in particular solar radiation.

27. Process for the protection of a photosensitive industrial material against the harmful effects of UV radiation, in particular solar radiation, which consists in incorporating, in the composition of the said material, an effective amount of a compound of formula (I) as defined in Claims 8 to 12.

28. Process for the protection of a photosensitive industrial material against the harmful effects of UV radiation, in particular solar radiation, which consists in applying, to the surface of the said material, an effective amount of a compound of formula (I) as defined in Claims 8 to 12.

## Patentansprüche

1. Aminosäurederivat, das der folgenden Formel (I) 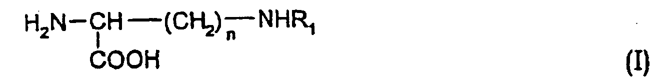 entspricht, in der bedeuten:
- n eine ganze Zahl von 1 bis 4,
- der Rest R₁ eine der Gruppen der folgenden Formeln (1), (2), (3), (4), (5), (6) oder (7) 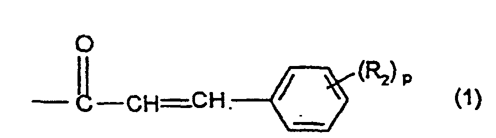 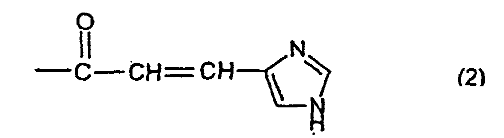 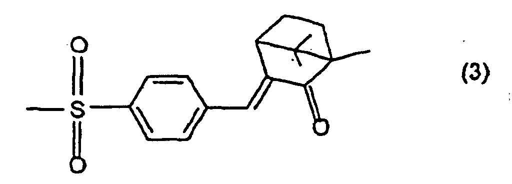 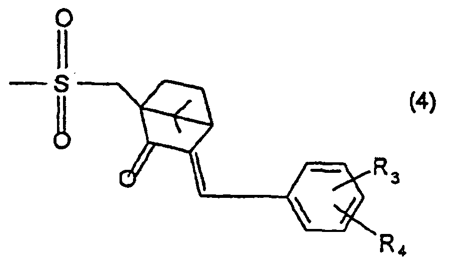 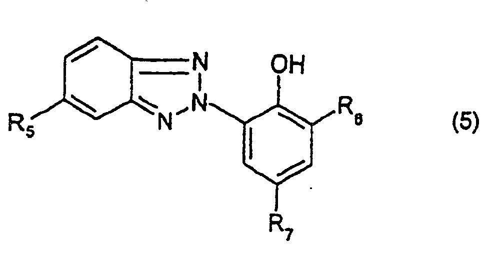 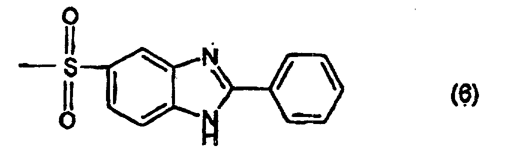 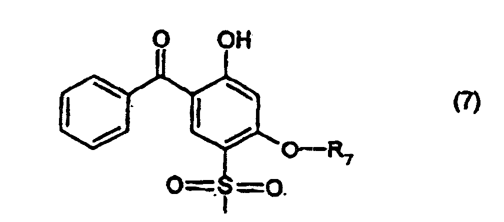
in denen bedeuten:
- p eine ganze Zahl von 1 bis 3;
- R₂ Wasserstoff, C₁₋₈-Alkyl, C₁₋₈-O-Alkyl, C₁₋₈-O-Acyl;
- R₃ und R₄, ähnlich oder verschieden, Wasserstoff oder ein Halogenatom oder C₁₋₈-Alkyl oder C₁₋₈-Alkoxy oder eine Gruppe der folgenden Formel (8) 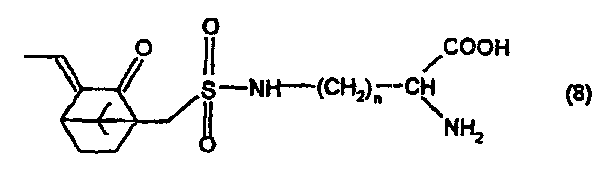
- R₅ Wasserstoff, ein Halogenatom, C₁₋₈-Alkyl oder C₁₋₈-Alkoxy oder einen zweiwertigen Rest 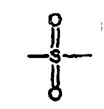
- R₆ Wasserstoff, C₁₋₈-Alkyl oder einen zweiwertigen Rest 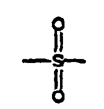 mit der Einschränkung, dass mindestens einer der beiden Substituenten R₅, R₆ 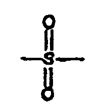 bedeutet,
- R₇ C₁₋₈-Alkyl;
sowie seine davon abgeleiteten Salze mit einer Säure oder einer Base,
wobei die Verbindung in Form eines Isomerengemischs oder in Form eines reinen Isomers vorliegt,
mit Ausnahme von N^{ε}-Cinnamoyl-L-lysin und 3-[(1-Oxo-3-phenyl-2-propenyl)-amino]-L-alanin und seines Monohydrochlorids.

2. Aminosäurederivat nach Anspruch 1, **dadurch gekennzeichnet, dass** es unter den Ornithinderivaten (n = 3) und den Lysinderivaten (n = 4) ausgewählt ist.

3. Aminosäurederivat nach Anspruch 1 oder 2, **dadurch gekennzeichnet, dass** es ausgewählt ist unter
- N^{ε}-(4-Methoxycinnamoyl)-L-lysin;
- N^{ε}-[4-(4,7,7-Trimethyl-3-oxo-bicyclo[2.2.1]hept-2-ylidenmethyl)-benzolsulfonyl]-L-lysin.

4. Aminosäurederivat nach einem der Ansprüche 1 bis 3, **dadurch gekennzeichnet, dass** es in Form von Partikeln vorliegt, die eine mittlere Größe unter 50 µm aufweisen.

5. Aminosäurederivat nach einem der Ansprüche 1 bis 4, **dadurch gekennzeichnet, dass** es in Form von Partikeln vorliegt, die eine mittlere Größe im Bereich von 0,01 µm bis etwa 20 µm aufweisen.

6. Verfahren zur Herstellung der Verbindungen der Formel (I), die wie in einem der Ansprüche 1 bis 5 definiert sind, wobei
(a) in einem ersten Schritt der Teil ₂HN-CH(COOH)- eines Monohydrochlorids einer Aminosäure der folgenden Formel (II) 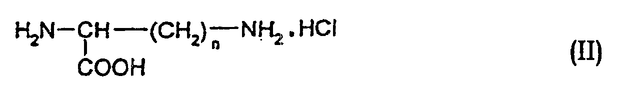 mit einem Kupfersulfat in einem basischen Medium in Gegenwart eines polaren Lösemittels komplexiert wird;
(b) in einem zweiten Schritt der so erhaltene Kupferkomplex in Gegenwart eines polaren organischen Lösemittels mit einem Säurechlorid R₁COCl oder einem Sulfonylchlorid R₁SO₂Cl umgesetzt wird, worin R₁ die gleiche Bedeutung hat, die in der Formel (I) angegeben ist, die in Anspruch 1 definiert ist;
(c) in einem dritten Schritt der in Schritt (b) erhaltene Niederschlag durch Filtration gewonnen wird,
(d) das so erhaltene Produkt mit einem Maskierungsmittel dekomplexiert wird.

7. Zusammensetzung, die dafür vorgesehen ist, ein gegenüber Ultraviolett-Strahlung, insbesondere Sonnenlicht, empfindliches Material zu schützen, **dadurch gekennzeichnet, dass** sie eine wirksame Menge mindestens einer Verbindung der folgenden Formel (I) 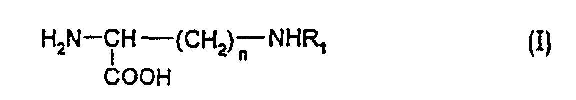 enthält, in der bedeuten:
- n eine ganze Zahl von 1 bis 4,
- der Rest R₁ eine der Gruppen der folgenden Formeln (1), (2), (3), (4), (5), (6) oder (7) 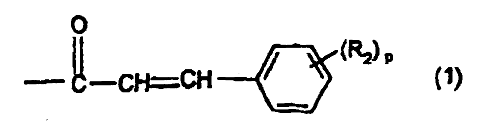 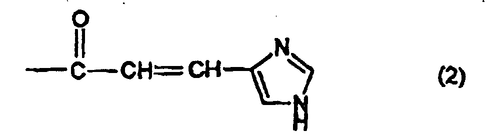 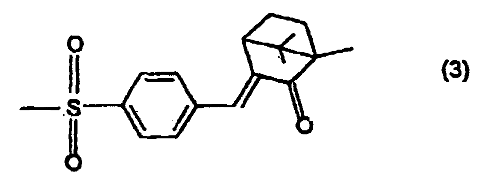 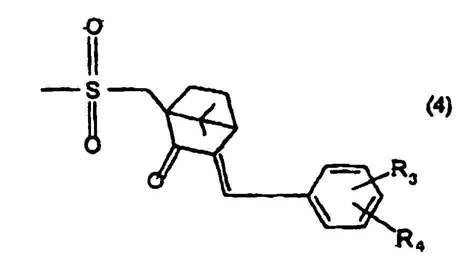 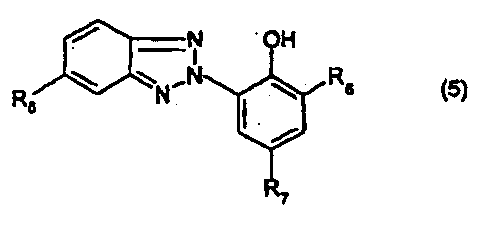 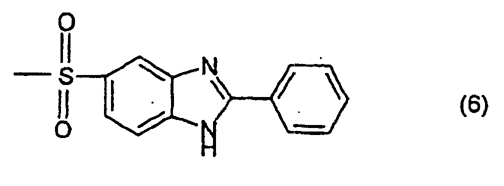 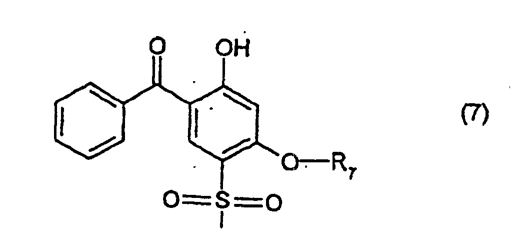
in denen bedeuten:
- p eine ganze Zahl von 1 bis 3;
- R₂ Wasserstoff, C₁₋₈-Alkyl, C₁₋₈-O-Alkyl, C₁₋₈-O-Acyl;
- R₃ und R₄, ähnlich oder verschieden, Wasserstoff oder ein Halogenatom oder C₁₋₈-Alkyl oder C₁₋₈-Alkoxy oder eine Gruppe der folgenden Formel (8) 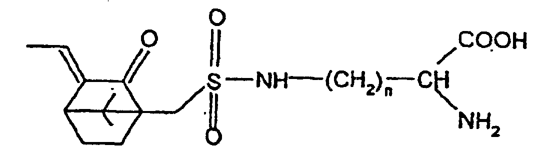
- Rs Wasserstoff, ein Halogenatom, C₁₋₈-Alkyl oder C₁₋₈-Alkoxy oder einen zweiwertigen Rest 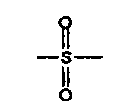
- R₆ Wasserstoff, C₁₋₈-Alkyl oder einen zweiwertigen Rest 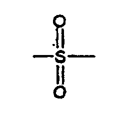 mit der Einschränkung, dass mindestens einer der beiden Substituenten R₅, R₆ 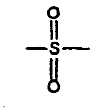 bedeutet,
- R₇ C₁₋₈-Alkyl;
sowie seine davon abgeleiteten Salze mit einer Säure oder einer Base,
wobei die Verbindung in Form eines Isomerengemischs oder in Form eines reinen Isomers vorliegt.

8. Kosmetische Zusammensetzung für den Lichtschutz der Haut und/oder der Haare, **dadurch gekennzeichnet, dass** sie in einem kosmetisch akzeptablen Träger eine wirksame Menge mindestens einer Verbindung der folgenden Formel (I) 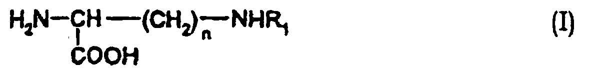 enthält, in der bedeuten:
- n eine ganze Zahl von 1 bis 4,
- der Rest R₁ eine der Gruppen der folgenden Formeln (1), (2), (3), (4), (5), (6) oder (7) 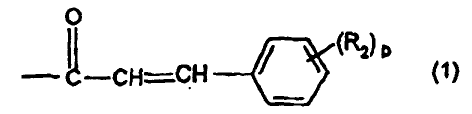 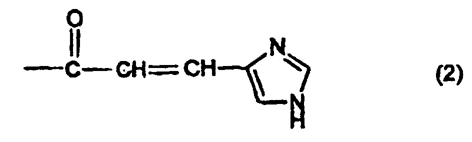 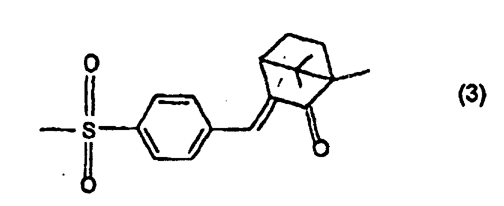 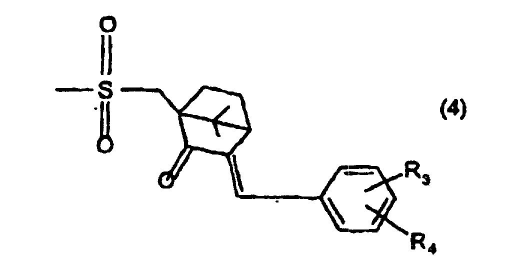 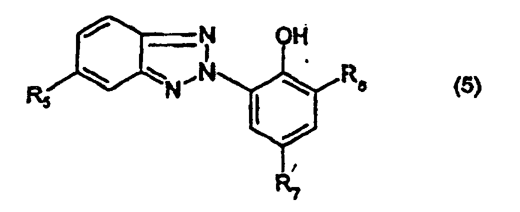 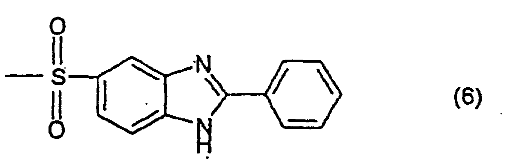 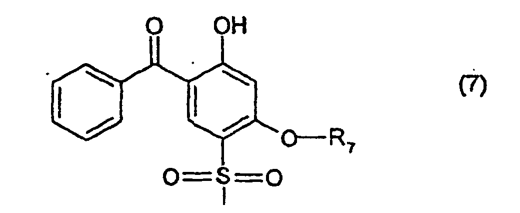
in denen bedeuten:
- p eine ganze Zahl von 1 bis 3;
- R₂ Wasserstoff, C₁₋₈-Alkyl, C₁₋₈-O-Alkyl, C₁₋₈-O-Acyl;
- R₃ und R₄, ähnlich oder verschieden, Wasserstoff oder ein Halogenatom oder C₁₋₈-Alkyl oder C₁₋₈-Alkoxy oder eine Gruppe der folgenden Formel (8) 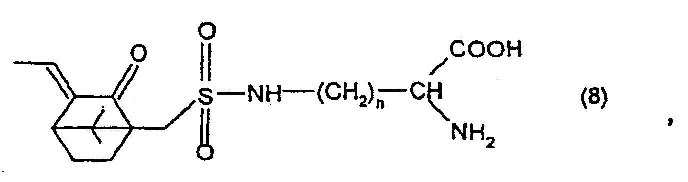
- R₅ Wasserstoff, ein Halogenatom, C₁₋₈-Alkyl oder C₁₋₈-Alkoxy oder einen zweiwertigen Rest 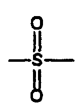
- R₆ Wasserstoff, C₁₋₈-Alkyl oder einen zweiwertigen Rest 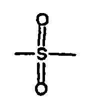 mit der Einschränkung, dass mindestens einer der beiden Substituenten R₅, R₆ 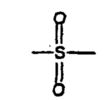 bedeutet,
- R₇ C₁₋₈-Alkyl;
sowie seine davon abgeleiteten Salze mit einer Säure oder einer Base,
wobei die Verbindung in Form eines Isomerengemischs oder in Form eines reinen Isomers vorliegt.

9. Zusammensetzung nach Anspruch 7 oder 8, **dadurch gekennzeichnet, dass** die Verbindung der Formel (I) unter den Ornithinderivaten (n = 3) und den Lysinderivaten (n = 4) ausgewählt ist.

10. Zusammensetzung nach einem der Ansprüche 7 bis 9, **dadurch gekennzeichnet, dass** die Verbindung der Formel (I) ausgewählt ist unter:
- N^{ε}-Cinnamoyl-L-lysin;
- N^{ε}-(4-Methoxycinnamoyl)-L-lysin;
- N^{ε}-[4-(4,7,7-Trimethyl-3-oxo-bicyclo[2.2.1]hept-2-ylidenmethyl)-benzolsulfonyl]-L-lysin.

11. Zusammensetzung nach einem der Ansprüche 7 bis 10, **dadurch gekennzeichnet, dass** die Verbindung der Formel (I) in Form von Partikeln vorliegt, die eine mittlere Größe unter 50 µm aufweisen.

12. Zusammensetzung nach einem der Ansprüche 7 bis 11, **dadurch gekennzeichnet, dass** die Verbindung der Formel (I) in Form von Partikeln vorliegt, die eine mittlere Größe im Bereich von 0,01 µm bis etwa 20 µm aufweisen.

13. Zusammensetzung nach einem der Ansprüche 8 bis 12, **dadurch gekennzeichnet, dass** sie 0,1 bis 20 Gew.-%, bezogen auf das Gesamtgewicht der Zusammensetzung, der Verbindung der Formel (I) enthält.

14. Zusammensetzung nach einem der Ansprüche 8 bis 13, **dadurch gekennzeichnet, dass** der kosmetisch akzeptable Träger in Form einer Emulsion vom Öl-in-Wasser-Typ vorliegt.

15. Zusammensetzung nach einem der Ansprüche 8 bis 14, **dadurch gekennzeichnet, dass** sie außerdem einen oder mehrere zusätzliche hydrophile oder lipophile organische Filter enthält, die im UV-Aund/ oder UV-B-Bereich wirksam sind.

16. Zusammensetzung nach Anspruch 15, **dadurch gekennzeichnet, dass** die zusätzlichen organischen Filter ausgewählt sind unter: den Zimtsäurederivaten, den Dibenzoylmethanderivaten; den Salicylsäurederivaten; den Campherderivaten; den Triazinderivaten; den Benzophenonderivaten; den β,β'-Diphenylacrylatderivaten, den Benzimidazolderivaten; den Bis-benzoazolylderivaten; den Bis-hydroxyphenolbenzotriazolderivaten; den p-Aminobenzoesäurederivaten; den Filtern aus Kohlenwasserstoffpolymeren und den Siliconfiltern.

17. Zusammensetzung nach einem der Ansprüche 8 bis 16, **dadurch gekennzeichnet, dass** sie außerdem als zusätzliche UV-Lichtschutzmittel Pigmente oder Nanopigmente von Metalloxiden, die umhüllt oder nicht umhüllt sind, enthält.

18. Zusammensetzung nach Anspruch 17, **dadurch gekennzeichnet, dass** die Pigmente oder Nanopigmente ausgewählt sind unter den Oxiden von Titan, Zink, Eisen, Zirconium, Cer und den Gemischen dieser Oxide, die umhüllt oder nicht umhüllt sind.

19. Zusammensetzung nach einem der Ansprüche 8 bis 18, **dadurch gekennzeichnet, dass** sie außerdem mindestens ein Mittel zum Bräunen und/oder Braunfärben der Haut enthält.

20. Zusammensetzung nach einem der Ansprüche 8 bis 19, **dadurch gekennzeichnet, dass** sie außerdem mindestens einen Hilfsstoff enthält, der unter Fettsubstanzen, organischen Lösemitteln, Verdickungsmitteln, reizlindernden bzw. beruhigenden Mitteln, Antioxidantien, Trübungsmitteln, Stabilisierungsmitteln, Emollientien, Hydroxysäuren, Antischaummitteln, Hydratisierungsmitteln, Vitaminen, Parfüms, Konservierungsmitteln, grenzflächenaktiven Stoffen, Füllstoffen, Maskierungsmitteln, Polymeren, Treibmitteln, alkalisch machenden Mitteln oder Säuerungsmitteln, Farbmitteln ausgewählt ist.

21. Zusammensetzung nach einem der Ansprüche 8 bis 20, **dadurch gekennzeichnet, dass** es sich um eine Zusammensetzung für den Schutz der menschlichen Epidermis oder eine Sonnenschutzzusammensetzung handelt und dass sie in Form einer Dispersion von nichtionischen Vesikeln, einer Emulsion, insbesondere einer Emulsion vom Öl-in-Wasser-Typ, einer Creme, einer Milch, eines Gels, einer Gelcreme, einer Suspension, einer Dispersion, eines Puders, eines Sticks, eines festen Stifts, eines Schaums oder eines Sprays vorliegt.

22. Zusammensetzung nach einem der Ansprüche 8 bis 21, **dadurch gekennzeichnet, dass** es sich um eine Zusammensetzung zum Schminken der Wimpern, der Augenbrauen oder der Haut handelt und dass sie in fester oder pastöser, wasserfreier oder wässriger Form, in Form einer Emulsion, einer Suspension oder einer Dispersion vorliegt.

23. Zusammensetzung nach einem der Ansprüche 8 bis 22, **dadurch gekennzeichnet, dass** es sich um eine Zusammensetzung handelt, die für den Schutz der Haare vor Ultraviolett-Strahlung vorgesehen ist, und dass sie in Form eines Haarwaschmittels, einer Lotion, eines Gels, einer Emulsion, einer Dispersion nicht-ionischer Vesikeln vorliegt.

24. Verwendung mindestens eine Verbindung der Formel (I), die wie in den Ansprüche 8 bis 12 definiert ist, für die Herstellung von Zusammensetzungen, die für den Schutz industrieller Materialien vorgesehen sind, die gegenüber Ultraviolett-Strahlung, insbesondere Sonnenlicht, empfindlich sind.

25. Verwendung nach Anspruch 24, wobei es sich bei den lichtempfindlichen industriellen Materialien um organische und/oder anorganische Gläser, Kunststoffmaterialien, Textilien, Anstrichmittel, Lacke, Lebensmittelprodukte handelt.

26. Verwendung mindestens einer Verbindung der Formel (I), die wie in einem der Ansprüche 8 bis 12 definiert ist, für die Herstellung von kosmetischen Zusammensetzungen für den Schutz der Haut und/oder der Haare vor Ultraviolett-Strahlung, insbesondere Sonnenlicht.

27. Verfahren zum Schützen eines lichtempfindlichen industriellen Materials vor den schädlichen Wirkungen von UV-Strahlung, insbesondere Sonnenlicht, das darin besteht, in die Zusammensetzung des Materials eine wirksame Menge einer Verbindung der Formel (I), die wie in den Ansprüchen 8 bis 12 definiert ist, einzubringen.

28. Verfahren zum Schützen eines lichtempfindlichen industriellen Materials vor den schädlichen Wirkungen von UV-Strahlung, insbesondere Sonnenlicht, das darin besteht, auf die Oberfläche des Materials eine wirksame Menge einer Verbindung der Formel (I), die wie in den Ansprüchen 8 bis 12 definiert ist, aufzutragen.
